# EUROPEAN PATENT APPLICATION

(11) **EP 4 498 388 A1**
(43) Date of publication of application: **29.01.2025**
(21) Application number: 23775281.1
(22) Date of filing: 21.03.2023
(51) Int. Cl.: G16H 50/20, G16H 80/00, G16H 30/40, A61B 3/14, G06N 20/20, G06N 3/045, G06N 3/08

(54) **DISEASE DIAGNOSIS METHOD USING TRAINED MODEL, AND SYSTEM CARRYING OUT SAME**

(30) Priority: 21.03.2022 KR 20220034781
(71) Applicant: Thyroscope Inc., Ulsan 44538 (KR)
(72) Inventor: SHIN, Kyubo, Ulju-gun Ulsan 44951 (KR); KIM, Jongchan, Ulsan 44469 (KR)
(74) Representative: Dantz, Jan Henning
(86) International application number: PCT/KR2023/003751
(87) International publication number: WO 2023/182785

(57) **Abstract**

Proposed is a sign prediction method including obtaining a facial image including eye region, obtaining a plurality of result values using the facial image and a diagnostic model for predicting an eye-related sign, wherein the plurality of result values include a plurality of predicted values and a single consensus predicted value, and determining a presence of the eye-related sign based on the consensus predicted value among the plurality of result values, wherein the diagnostic model is generated using training data in which a plurality of determination values, independently determined by two or more different diagnosticians with respect to a presence of the eye-related sign in a same diagnostic subject, and a single consensus value, determined by the diagnosticians through mutual agreement with respect to a presence of the eye-related sign in the same diagnostic subject, are multi-labeled on one diagnostic image, and the plurality of predicted values correspond to the plurality of determination values, and the consensus predicted value corresponds to the consensus value.

## Description

### Technical Field

The present disclosure relates to a disease diagnosis method using a trained model, and a system carrying out the same.

### Background Art

In the medical field, trained models used for disease diagnosis have been actively developed.

Due to the characteristics of disease diagnosis, even for the same diagnostic subject, diagnostic results may vary depending on a health care provider's rule of thumb or inclination.

Therefore, it is a well-known popular opinion that in generating a trained model for disease diagnosis, if a diagnostic model is trained simply using only information on a diagnostic subject and information on a diagnostic result as training data without distinguishing between health care providers, health care providers' different rules of thumb or inclinations applied to diagnostic results are diluted during the training process and the accuracy of the trained model is decreased.

Therefore, in the field of disease diagnosis using trained models, rather than generating diagnostic models without distinguishing between health care providers, systems have been developed that generate a diagnostic model for each health care provider and select a value determined by the majority of result values of the respective diagnostic models as a finally predicted disease diagnosis value if necessary.

However, the accuracy of artificial intelligence models cannot be 100 %, and a system for predicting a disease diagnosis value by using result values of respective diagnostic models cannot guarantee the accuracy of final disease diagnosis because many inaccurate predicted values are used and inaccuracies are accumulated.

Accordingly, it is necessary to develop a method to obtain a final diagnosis value with reduced accuracy loss and to apply diagnostic results respectively determined by a plurality of health care providers.

### Disclosure

### Technical Problem

The disclosure in the present application is directed to providing a trained model capable of reducing accuracy loss while considering determination values for respective diagnosticians.

In addition, the disclosure in the present application is directed to providing a trained model capable of predicting a disease by using an image obtained with a digital camera that ordinary people can use, rather than a professional medical diagnostic device.

In addition, the disclosure in the present application is directed to providing a system enabling ordinary people to monitor a clinical activity score for thyroid eye disease without a doctor's help and an in-person visit to a hospital.

Technical problems to be solved by the present application are not limited to the aforementioned technical problems and other technical problems which are not mentioned will be clearly understood by those skilled in the art from the present specification and the accompanying drawings.

### Technical Solution

According to an embodiment of the present application, there is provided a sign prediction method including: obtaining a facial image including eye region; obtaining a plurality of result values using the facial image and a diagnostic model for predicting an eye-related sign, wherein the plurality of result values include a plurality of predicted values and a single consensus predicted value; and determining a presence of the eye-related sign based on the consensus predicted value among the plurality of result values, wherein the diagnostic model is generated using training data in which a plurality of determination values, independently determined by two or more different diagnosticians with respect to a presence of the eye-related sign in a same diagnostic subject, and a single consensus value, determined by the diagnosticians through mutual agreement with respect to a presence of the eye-related sign in the same diagnostic subject, are multi-labeled on one diagnostic image, wherein the plurality of predicted values corresponds to the plurality of determination values, and the consensus predicted value corresponds to the consensus value, wherein the eye-related sign is at least one of a conjunctival hyperemia, a conjunctival edema, a lacrimal edema, an eyelid redness and an eyelid edema.

According to an embodiment of the present application, at least some of the training data may have the consensus value corresponding to a minority of the determination values among the plurality of determination values.

According to an embodiment of the present application, when training the diagnostic model, a loss weight for a node where the consensus predicted value is output may be set higher than a loss weight for each node where the predicted values are output.

According to an embodiment of the present application, obtaining the plurality of result values may include: performing preprocessing on the facial image for the eye region; and obtaining the plurality of result values using the preprocessed image and the diagnostic model.

According to an embodiment of the present application, performing preprocessing may include: when the eye-related sign is at least one of the conjunctival hyperemia, the conjunctival edema, and the lacrimal edema, performing preprocessing on the facial image to mask a pupil and skin; and when the eye-related sign is at least one of the eyelid redness and the eyelid edema, performing preprocessing on the facial image to mask an eyeball region.

According to an embodiment of the present application, the diagnosticians may be three ophthalmologists with over 15 years of experience, wherein the determination values may include three values corresponding to each diagnostician.

According to an embodiment of the present application, at least one of the determination values and the consensus value may be a value determined by the diagnosticians through actually meeting with the diagnostic subject with respect to the presence of the eye-related sign.

According to an embodiment of the present application, at least one of the determination values and the consensus value may be a value determined by the diagnosticians through a facial image of the diagnostic subject with respect to the presence of the eye-related sign.

According to an embodiment of the present application, the consensus value may be determined after the diagnosticians determine each of the determination values.

According to an embodiment of the present application, the diagnostic model may be generated using at least one algorithm selected from a group of a convolution neural network (CNN), a vision transformer, a support vector machine (SVM), a random forest, a gradient boosting algorithm, an artificial neural network (ANN), a deep neural network (DNN), a recurrent neural network (RNN), ResNet, VGG, GoogLeNet, and MobileNet.

However, the solving means of the problems of the present disclosure are not limited to the aforementioned solving means and other solving means which are not mentioned will be clearly understood by those skilled in the art from the present specification and the accompanying drawings.

### Advantageous Effects

According to the disclosure in the present application, by using training data in which determination values of a plurality of diagnosticians and a consensus value of the diagnosticians are multi-labeled on a diagnostic image, a diagnostic model capable of reducing accuracy loss while considering the respective determination values of the plurality of diagnosticians can be generated.

According to the disclosure in the present application, by using training data in which a label value determined by applying determination values of a plurality of diagnosticians to a consensus value of the diagnosticians is labeled on a diagnostic image, a diagnostic model capable of reducing accuracy loss while considering the respective determination values of the plurality of diagnosticians can be generated.

According to the disclosure in the present application, a diagnostic model capable of predicting the presence of a disease by using an image obtained with a digital camera that ordinary people can use, rather than a professional medical diagnostic device can be generated.

According to the disclosure in the present application, a system enabling ordinary people to monitor a clinical activity score for thyroid eye disease without a doctor's help and an in-person visit to a hospital can be provided.

### Description of Drawings

FIG. 1 is a diagram illustrating a system for diagnosing a disease according to an embodiment.
FIG. 2 is a block diagram illustrating a configuration of a user device according to an embodiment.
FIG. 3 is a diagram illustrating a configuration of a server according to an embodiment.
FIG. 4 is a diagram illustrating a process of obtaining a determination value for each diagnostician according to an embodiment.
FIG. 5 is a diagram illustrating a process of obtaining a consensus value from a plurality of diagnosticians according to an embodiment.
FIG. 6 is a diagram illustrating training data used to train a diagnostic model according to an embodiment.
FIG. 7 is a diagram illustrating a process of predicting a disease by using a trained diagnostic model according to an embodiment.
FIG. 8 is a diagram illustrating training data used to train a diagnostic model according to an embodiment.
FIG. 9 is a diagram illustrating a process of predicting a disease by using a trained diagnostic model according to an embodiment.
FIG. 10 is a diagram illustrating training data used to train a diagnostic model according to an embodiment.
FIG. 11 is a diagram illustrating a process of predicting a disease by using a trained diagnostic model according to an embodiment.
FIG. 12 is a diagram illustrating a system for predicting a clinical activity score for thyroid eye disease according to an embodiment.
FIGS. 13 and 14 are diagrams illustrating an image preprocessing process according to an embodiment.

### Best Mode

Embodiments described in the present specification are for clearly describing the idea of the present disclosure to those skilled in the art to which the present disclosure belongs, so the present disclosure is not limited to the embodiments described in the present specification and the scope of the present disclosure should be construed as including modifications or variations that are within the idea of the present disclosure.

As the terms used in the present specification, general terms currently widely used are used considering functions in the present disclosure. However, the terms may vary according to the intentions of those skilled in the art, customs, or the emergence of new technology. However, unlike this, when a particular term is used defined as having an optional meaning, the meaning of the term will be described. Thus, the terms used in the present specification should be construed based on the actual meanings of the terms and details throughout the present specification rather than simply the names of the terms.

Numbers (for example, first, second, etc.) used in the description of the present specification are merely identifiers for distinguishing one element from another.

In the following embodiments, an expression used in the singular encompasses the expression of the plural, unless it has a clearly different meaning in the context.

In the following embodiments, it is to be understood that terms such as "including" or "having" are intended to indicate the existence of features or elements disclosed in the specification, and are not intended to preclude the possibility that one or more other features or elements may be added.

The drawings accompanying the present specification are for easily describing the present disclosure, and the shapes shown in the drawings may be exaggerated to help the understanding of the present disclosure, so the present disclosure is not limited by the drawings.

In a case in which a particular embodiment is realized otherwise, a particular process may be performed out of the order described. For example, two processes described in succession may be performed substantially simultaneously, or may be performed in an order opposite to the order described.

In the present specification, if it is decided that a detailed description of known configuration or function related to the present disclosure makes the subject matter of the present disclosure unclear, the detailed description is omitted.

According to an embodiment of the present disclosure, there is provided a sign prediction method including: obtaining a facial image including eye region; obtaining a plurality of result values using the facial image and a diagnostic model for predicting an eye-related sign, wherein the plurality of result values include a plurality of predicted values and a single consensus predicted value; and determining a presence of the eye-related sign based on the consensus predicted value among the plurality of result values, wherein the diagnostic model is generated using training data in which a plurality of determination values, independently determined by two or more different diagnosticians with respect to a presence of the eye-related sign in a same diagnostic subject, and a single consensus value, determined by the diagnosticians through mutual agreement with respect to a presence of the eye-related sign in the same diagnostic subject, are multi-labeled on one diagnostic image, wherein the plurality of predicted values corresponds to the plurality of determination values, and the consensus predicted value corresponds to the consensus value, wherein the eye-related sign is at least one of a conjunctival hyperemia, a conjunctival edema, a lacrimal edema, an eyelid redness and an eyelid edema.

According to an embodiment of the present application, at least some of the training data may have the consensus value corresponding to a minority of the determination values among the plurality of determination values.

According to an embodiment of the present application, when training the diagnostic model, a loss weight for a node where the consensus predicted value is output may be set higher than a loss weight for each node where the predicted values are output.

According to an embodiment of the present application, obtaining the plurality of result values may include: performing preprocessing on the facial image for the eye region; and obtaining the plurality of result values using the preprocessed image and the diagnostic model.

According to an embodiment of the present application, performing preprocessing may include: when the eye-related sign is at least one of the conjunctival hyperemia, the conjunctival edema, and the lacrimal edema, performing preprocessing on the facial image to mask a pupil and skin; and when the eye-related sign is at least one of the eyelid redness and the eyelid edema, performing preprocessing on the facial image to mask an eyeball region.

According to an embodiment of the present application, the diagnosticians may be three ophthalmologists with over 15 years of experience, wherein the determination values may include three values corresponding to each diagnostician.

According to an embodiment of the present application, at least one of the determination values and the consensus value may be a value determined by the diagnosticians through actually meeting with the diagnostic subject with respect to the presence of the eye-related sign.

According to an embodiment of the present application, at least one of the determination values and the consensus value may be a value determined by the diagnosticians through a facial image of the diagnostic subject with respect to the presence of the eye-related sign.

According to an embodiment of the present application, the consensus value may be determined after the diagnosticians determine each of the determination values.

According to an embodiment of the present application, the diagnostic model may be generated using at least one algorithm selected from a group of a convolution neural network (CNN), a vision transformer, a support vector machine (SVM), a random forest, a gradient boosting algorithm, an artificial neural network (ANN), a deep neural network (DNN), a recurrent neural network (RNN), ResNet, VGG, GoogLeNet, and MobileNet.

Hereinafter, a diagnostic system and a method of generating a diagnostic model according to an embodiment will be described.

### 1. The configuration of a diagnostic system 10

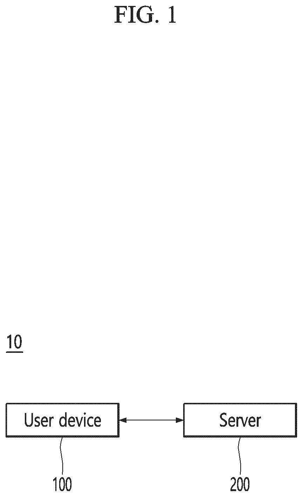

FIG. 1 is a diagram illustrating a diagnostic system 10 for diagnosing a disease according to an embodiment.

A diagnostic system 10 according to an embodiment may perform a disease diagnosis using information obtained targetting a person. The information obtained targetting the person may be various types of information. For example, the information obtained targetting the person may be various types of information, such as an image of all or part of the person's body, sound generated from the person, a biometric signal of the person, information related to the person's biological response, and information related to the person's behavior.

Accordingly, for example, the diagnostic system 10 may predict the presence of a disease using an image taken of at least part of a user's body and a trained diagnostic model. As another example, the diagnostic system 10 may predict the presence of a disease using sound generated from a user's body and a trained diagnostic model. As a specific example, the diagnostic system 10 may predict the presence of an eye disease using a facial image taken of a user's face and a trained eye disease diagnostic model, and is not limited thereto.

Hereinafter, for convenience of description, the diagnostic system 10 will be described assuming that a disease is diagnosed on the basis of an image.

Referring to FIG. 1, the diagnostic system 10 may include a user device 100 and a server 200.

The user device 100 is a device for interacting directly and/or indirectly with a user. The user device 100 may generate a diagnostic image by photographing a diagnostic subject that is at least part of a user's body.

The user device 100 may transmit a diagnostic image of a diagnostic subject to the server 200, which will be described later. Specifically, the user device 100 may transmit a diagnostic image to the server 200 through a wired and/or wireless data communication method, or may transmit a diagnostic image to the server 200 through other external devices.

The user device 100 may receive information related to a diagnostic result from the server 200. The user device 100 may provide information obtained from the server 200 to the user in a visual and/or audible manner.

For example, the user device 100 may include a user input device and/or a photographing device, such as a smartphone, a tablet computer, a desktop, a laptop computer, and a digital camera.

The server 200 may use a trained diagnostic model to predict the presence of a disease and/or a sign in a diagnostic image. The diagnostic image may be directly received from the user device 100, or may be received through an external device, such as a relay device and/or another external server.

The server 200 may transmit a prediction result to the user device 100 and/or other external devices.

The server 200 may obtain a trained diagnostic model from an external device and store the trained diagnostic model. Without being limited thereto, the server 200 may generate a diagnostic model using a diagnostic image in which a label value is labeled, as training data.

In the meantime, the server 200 and the user device 100 have been described as being distinguished from each other, but the server 200 and the user device 100 may be realized as a single device.

### 2. The configuration of a user device 100

Hereinafter, a configuration of a user device 100 in the configuration of a diagnostic system 10 will be described.

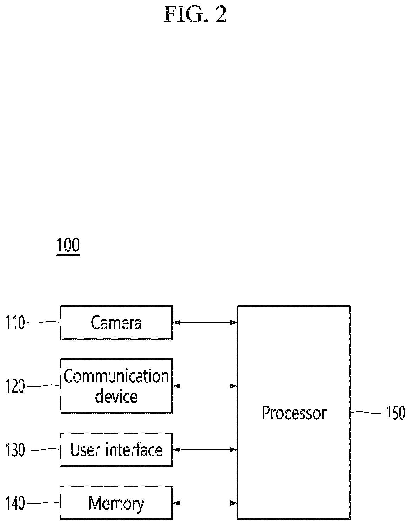

FIG. 2 is a block diagram illustrating a configuration of a user device 100 according to an embodiment.

Referring to FIG. 2, the user device 100 may include a camera 110, a communication device 120, a user interface 130, a memory 140, and a processor 150.

The camera 110 is a digital camera, and may include an image sensor and an image processor. The image sensor is a device for converting an optical image into electrical signals, and may be provided as a chip in which multiple photodiodes are integrated. Examples of the image sensors may include a charge-coupled device (CCD), and a complementary metal-oxide-semiconductor (CMOS). In the meantime, the image processor may perform image processing on captured results, and may generate image information.

The communication device 120 may transmit or receive data and/or information to or from the outside through wired and/or wireless communication. The communication device 120 may perform bi-directional or uni-directional communication.

The communication device 120 may include a wireless communication module and/or a wired communication module. Herein, examples of the wireless communication modules may include a Wi-Fi communication module, and a cellular communication module.

The user interface 130 may output various types of information according to control commands of the processor 150. According to an embodiment, the user interface 130 may include a display for outputting information visually to a user. The user interface 130 may include a speaker for outputting information audibly to a user. The user interface 130 may include a vibration motor for outputting information tactually to a user.

In the meantime, the user device 100 may receive various types of information required for the operation of the user device 100 from a user through the user interface 130. For example, the user device 100 may receive information related to a disease and/or a sign from a user through the user interface 130, but is not limited thereto.

The memory 140 may store various processing programs, parameters for performing processing of the programs, or data resulting from such processing. For example, the memory 140 may store instructions for the operation of the processor 150, which will be described later, various preprocessing algorithms for diagnostic images and/or executable codes for realizing trained models. Furthermore, the memory 140 may store a diagnostic image obtained through the camera 110, and a preprocessed diagnostic image.

The memory 140 may be realized as a non-volatile semiconductor memory, a hard disk drive (HDD), a solid-state disk (SSD), a silicon disk drive (SDD), a flash memory, a random-access memory (RAM), a read-only memory (ROM), an electrically erasable programmable read-only memory (EEPROM), or other types of tangible non-volatile recording media.

The processor 150 may control the overall operation of the user device 100, and may operate according to the instructions stored in the memory 140.

Specifically, the processor 150 may control the camera 110 to obtain a diagnostic image and store the diagnostic image in the memory 140. Examples of the diagnostic images may include any images related to a patient, such as the patient's body parts, a facial image, an eye image, and a full body image.

The processor 150 may process a diagnostic image using a preprocessing algorithm and/or a trained model stored in the memory 140. The processor 150 may use different preprocessing algorithms and/or trained models depending on a diagnostic subject, a type of a disease, and a type of a sign. For example, when a sign is related to an eye, the processor 150 may perform preprocessing, such as cropping and/or masking a portion of an image, to make the eye part more clearly visible. Without being limited thereto, the processor 150 may perform various types of preprocessing, such as color correction and/or brightness correction of an image.

The processor 150 may transmit an image and/or a preprocessed image to the server 200 and/or an external device through the communication device 120.

The processor 150 may provide information received from the server 200 and/or an external device to a user through an output device 130.

The processor 150 may be realized as a central processing unit (CPU), a graphics processing unit (GPU), a digital signal processor (DSP), a state machine, an application specific integrated circuit (ASIC), a radio-frequency integrated circuit (RFIC), and a combination thereof.

### 3. The configuration of a server 200

Hereinafter, a configuration of a server 200 in the configuration of a diagnostic system 10 will be described.

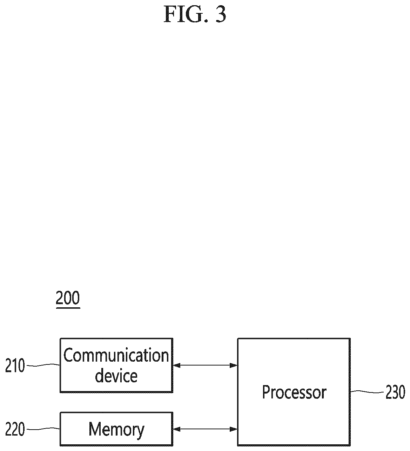

FIG. 3 is a diagram illustrating a configuration of a server 200 according to an embodiment.

Referring to FIG. 3, the server 200 may include a communication device 210, a memory 220, and a processor 230.

The communication device 210 may transmit and/or receive data and/or information to or from the outside through wired and/or wireless communication. The communication device 210 may perform bi-directional or uni-directional communication.

The communication device 210 may include a wireless communication module and/or a wired communication module. Herein, examples of the wireless communication modules may include a Wi-Fi communication module, and a cellular communication module.

The memory 220 may store various processing programs, parameters for performing processing of the programs, parameters for performing such processing, or data resulting from such processing. For example, the memory 220 may store instructions for the operation of the processor 230, which will be described later, various preprocessing algorithms for diagnostic images and/or executable codes for realizing trained models. Examples of the preprocessing algorithm and/or the trained model may include various preprocessing algorithms and/or trained models depending on a diagnostic subject and/or a disease.

The memory 220 may store a diagnostic image received from the user device 100 and/or an external device. The received diagnostic image may be a preprocessed image.

Examples of a trained model stored in the memory 220 may include a diagnostic model for predicting the presence of a disease and/or a sign in a diagnostic image. The memory 220 may include various types of diagnostic models for respective diagnostic subjects and/or diagnosis diseases. Further, the memory 220 may store various types of diagnostic models for the same disease. For example, a plurality of diagnostic models for diagnosing the presence of conjunctival hyperemia may be stored. The respective diagnostic models may be diagnostic models generated using different algorithms and/or training data. The diagnostic models stored in the memory 220 may be diagnostic models received from the outside, or may be diagnostic models generated by the server 200.

The memory 220 may store training data and a model generation algorithm used to generate a diagnostic model. The specific details of the training data will be described later.

The memory 220 may be realized as a non-volatile semiconductor memory, a hard disk drive (HDD), a solid-state disk (SSD), a silicon disk drive (SDD), a flash memory, a random-access memory (RAM), a read-only memory (ROM), an electrically erasable programmable read-only memory (EEPROM), or other types of tangible non-volatile recording media.

The processor 230 may control the overall operation of the server 200, and may operate according to the instructions stored in the memory 220.

The processor 230 may use a diagnostic model stored in the memory 220 to predict whether a diagnostic subject shown in a diagnostic image has a disease and/or a sign.

The processor 230 may apply different diagnostic models to one diagnostic image, thereby generating various types of prediction results. For example, the processor 230 may use, for one eye image, respective diagnostic models for diagnosing conjunctival hyperemia, conjunctival edema, eyelid redness, eyelid edema, and lacrimal edema to generate various types of prediction results, such as the presence of conjunctival hyperemia, the presence of conjunctival edema, the presence of eyelid redness, the presence of eyelid edema, and the presence of lacrimal edema.

Without being limited thereto, the processor 230 may apply one diagnostic model for predicting a plurality of types of diseases and/or signs for one diagnostic image, thereby generating various types of prediction results. For example, the processor 230 may use, for one eye image, one diagnostic model for diagnosing at least two selected from the group of conjunctival hyperemia, conjunctival edema, eyelid redness, eyelid edema, and lacrimal edema and may generate at least two prediction results among the presence of conjunctival hyperemia, the presence of conjunctival edema, the presence of eyelid redness, the presence of eyelid edema, and the presence of lacrimal edema.

The processor 230 may determine, on the basis of a diagnostic image, a diagnostic model to be used for prediction. For example, when the processor 230 determines that a diagnostic image is a facial image, the processor 230 may determine a diagnostic model for predicting a face-related disease and/or sign and may use the determined diagnostic model to predict a disease and/or a sign.

The processor 230 may perform preprocessing on a diagnostic image before using a diagnostic model. The processor 230 may perform preprocessing on a diagnostic image by using different preprocessing algorithms depending on a diagnostic subject and/or a diagnosis disease. For example, when a diagnostic subject and/or a diagnosis disease is related to a user's face, the processor 230 may crop a diagnostic image to leave only a face region. When a diagnostic subject and/or a diagnosis disease is related to a user's eye, the processor 230 may crop a diagnostic image to leave only an eye region.

The processor 230 may generate a diagnostic result on the basis of a prediction result obtained using a diagnostic model. For example, the processor 230 may use respective diagnostic models for diagnosing conjunctival hyperemia, conjunctival edema, eyelid redness, eyelid edema, and lacrimal edema for an eye image to obtain respective prediction results for the presence of conjunctival hyperemia, the presence of conjunctival edema, the presence of eyelid redness, the presence of eyelid edema, and the presence of lacrimal edema, and may generate, on the basis of the obtained prediction results, a diagnostic result including a clinical activity score (CAS) for thyroid eye disease.

The processor 230 may transmit the presence of a disease and/or a diagnostic result to the user device 100 and/or an external device through the communication device 210.

The processor 230 may generate a diagnostic model using training data and a model generation algorithm stored in the memory 220. The details related to the generation of a diagnostic model using training data will be described later.

The processor 230 may be realized as a central processing unit (CPU), a graphics processing unit (GPU), a digital signal processor (DSP), a state machine, an application specific integrated circuit (ASIC), a radio-frequency integrated circuit (RFIC), and a combination thereof.

### 4. Diagnostic model 1 - multi-label model

Hereinafter, the specific details of a multi-label model for diagnosing a disease and/or a sign will be described.

A multi-label model according to an embodiment may be a model generated using training data in which a plurality of determination values respectively determined by a plurality of diagnosticians and one consensus value are multi-labeled on one diagnostic image.

A diagnostic image may mean an image of a diagnostic subject. For example, a diagnostic image may be an image of all or part of a user's body obtained by the user using a user device. Without being limited thereto, a diagnostic image may mean various types of images, such as a camera image, an X-ray image, a computed tomography (CT) image, and magnetic resonance imaging (MRI), of a subject to be diagnosed.

A diagnostic subject may mean an analysis subject to determine a user's state related to a disease and/or a sign. For example, the diagnostic subject may be various types of subjects, such as a user's arms, legs, face, torso, and internal organs. A diagnostic subject may be more specific, and may be determined to be various and specific, for example, the cornea, eyelid, and iris of the eye on the face, depending on a disease to be diagnosed.

A diagnostician may mean a person who derives information related to a disease and/or a sign in a diagnostic image. For example, a diagnostician may be a medical staff or a clinical manager, but is not limited thereto. There may be at least two diagnosticians, preferably, three diagnosticians, who derive information from one diagnostic image.

Diagnosticians may be determined among the people with a particular level or more of experience, depending on a diagnostic subject and/or a diagnosis disease. For example, it was found through investigation that in the field of eye disease diagnosis, the average diagnosis accuracy of ophthalmologists with over 15 years of experience was about 86.24 %, the average diagnosis accuracy of ophthalmology fellows with less than 5 years of experience was about 73.27 %, and the average accuracy of ophthalmology residents was 56.04 %. Accordingly, it is preferable that a diagnostician who derives information related to a disease and/or a sign to be used to train a diagnostic model with an eye disease as a diagnosis sign is determined as an ophthalmologist with over 15 years of experience to ensure the accuracy of the diagnostic model.

A determination value may mean information on the presence of a disease and/or a sign determined by an individual diagnostician analyzing a diagnostic image independently. For example, when a diagnostic image is an eye image and a target disease is conjunctival hyperemia, a determination value may be information on the presence of conjunctival hyperemia.

A determination value may be expressed as a positive or a negative for a disease and/or a sign, but is not limited thereto. A determination value may be expressed as the presence/absence of a disease, as true or false, as 1 or 0, or as one of the values distinguished by a plurality of classes.

A determination value is determined by a diagnostician analyzing a diagnostic image independently, so determination values may differ between diagnosticians.

In the meantime, a determination value may be determined by a diagnostician through a meeting with an actual user. Specifically, a diagnostician may determine a determination value by checking an actual user with the naked eye. In this case, a diagnostic image may be obtained from a user after a diagnostician determines a determination value, or may be obtained from a user before a diagnostician meets the actual user.

A consensus value may mean information on the presence of a disease and/or a sign determined by a plurality of diagnosticians analyzing a diagnostic image together and reaching an agreement. For example, a consensus value may be a value determined by a plurality of diagnosticians discussing the same diagnostic image with each other at the same time and/or in the same space.

A plurality of diagnosticians determining a consensus value and the diagnosticians determining determination values may mean the same diagnosticians. That is, a plurality of diagnosticians may analyze a diagnostic image independently of each other to determine determination values, and then reach a mutual agreement to determine one consensus value.

A consensus value may be expressed in the same form as a determination value. For example, a consensus value may be expressed as a positive or a negative for a disease and/or a sign, but is not limited thereto. A consensus value may be expressed as the presence/absence of a disease, as true or false, as 1 or 0, or as one of the values distinguished by a plurality of classes.

In the meantime, a consensus value may be determined by a plurality of diagnosticians through a meeting with an actual user. Specifically, a plurality of diagnosticians may determine a consensus value by checking an actual user with the naked eye and having a discussion. In this case, a plurality of diagnosticians may analyze an actual user independently of each other to determine respective determination values, and then have a discussion with each other and reach an agreement to determine a consensus value. In this case, a diagnostic image may be obtained from a user after a plurality of diagnosticians determines a consensus value, or may be obtained from a user before a plurality of diagnosticians meet the actual user.

A label value means a value labeled on a diagnostic image, in generating training data used to generate a diagnostic model. That is, training data may include a diagnostic image as training input data, and may include a label value as training result data.

A label value may be a determination value and/or a consensus value itself. Without being limited thereto, a label value may be a value determined on the basis of a determination value and/or a consensus value. For example, a determination value and/or a consensus value is a value indicating a positive, a label value may be a value of 1. When a determination value and/or a consensus value is a value indicating a negative, a label value may be a value of 0. A specific label value is not limited thereto, and may be determined a variety of ways depending on the algorithm design of a diagnostic model.

In the meantime, a person determining a label value may be different from a diagnostician. For example, after diagnosticians derive determination values and a consensus value for a diagnostic image, an artificial-intelligence engineer may determine label values on the basis of the determination values and the consensus value of the diagnosticians. As another example, when diagnosticians transmit determination values and a consensus value to a server, the server may determine label values on the basis of the determination values and the consensus value and may perform labelling on the diagnostic image.

The number of determination values and a consensus value may be at least three, so as training data to be used to generate a diagnostic model, at least three label values may be multi-labeled on the diagnostic image.

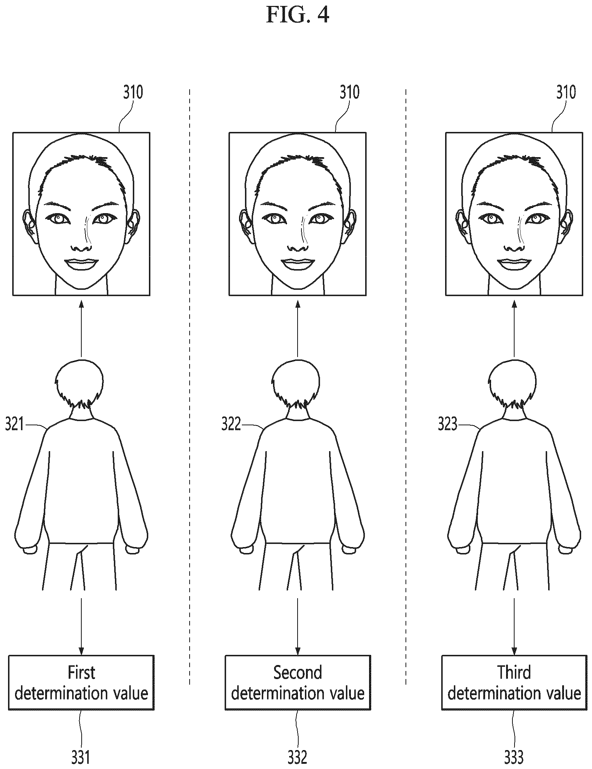

Training data used to generate a diagnostic model will be described with reference to FIGS. 4 to 6.

FIG. 4 is a diagram illustrating a process of obtaining a determination value for each diagnostician according to an embodiment.

Referring to FIG. 4, with respect to the same diagnostic image 310, a plurality of diagnosticians 321, 322, and 323 diagnose a state related to a disease and/or a sign independently of each other to determine respective determination values 331, 332, and 333. The determination values 331, 332, and 333 may be different values as they are values determined by the plurality of diagnosticians 321, 322, and 323 independently of each other. For example, a first diagnostician 321 and a second diagnostician 322 may determine a positive, and a third diagnostician 323 may determine a negative.

In the meantime, FIG. 4 shows three diagnosticians 321, 322, and 323, but no limitation thereto is imposed, and there may be two or four or more diagnosticians.

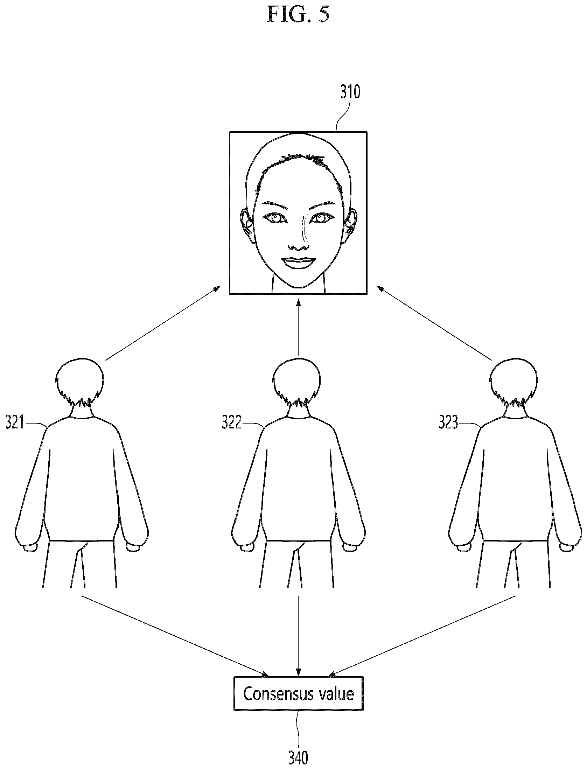

FIG. 5 is a diagram illustrating a process of obtaining a consensus value from a plurality of diagnosticians according to an embodiment.

Referring to FIG. 5, a plurality of diagnosticians 321, 322, and 323 reach a mutual agreement about a state related to a disease and/or a sign in a diagnostic image 310, thereby determining one consensus value 340.

The plurality of diagnosticians 321, 322, and 323 diagnosing the diagnostic image 310 and determining the consensus value 340 in FIG. 5 may be the same as the plurality of diagnosticians 321, 322, and 323 diagnosing the diagnostic image 310 independently and determining the determination values 331, 332, and 333 in FIG. 4.

The diagnostic image 310 diagnosed by the plurality of diagnosticians 321, 322, and 323 in FIG. 5 may be the same as the diagnostic image 310 used by the diagnosticians 321, 322, and 323 to determine the respective determination values 331, 332, and 333 in FIG. 4.

That is, with respect to the same diagnostic image 310, the diagnosticians 321, 322, and 323 may independently determine the respective determination values 331, 332, and 333 for the presence of a disease, and the same diagnosticians 321, 322, and 323 may determine the consensus value 340 by reaching an agreement about the presence of a disease with respect to the same diagnostic image 310. For example, in determining the consensus value 340, the diagnosticians 321, 322, and 323 may reach a mutual agreement at the same time and/or in the same space. Specifically, the consensus value 340 may be determined by the majority of the diagnosticians 321, 322, and 323, or the consensus value 340 may be determined on the basis of minority judgement through a discussion and an agreement among the diagnosticians 321, 322, and 323. As another example, the consensus value 340 may be determined by the majority of the determination values 331, 332, and 333 of the diagnosticians 321, 322, and 323.

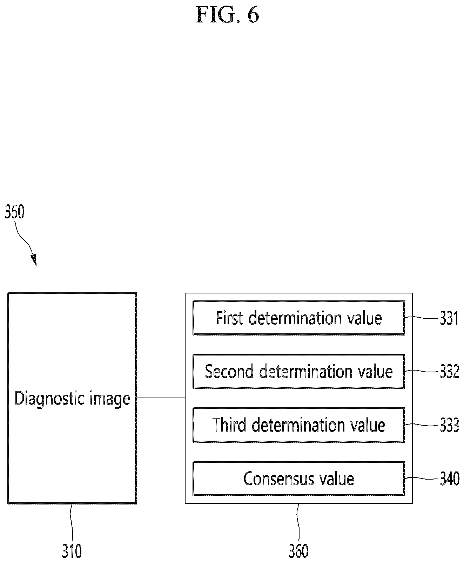

FIG. 6 is a diagram illustrating training data used to train a diagnostic model according to an embodiment.

Referring to FIG. 6, training data 350 used to generate a diagnostic model may include a diagnostic image 310, and a plurality of determination values 331, 332, and 333 and a consensus value 340 labeled on the diagnostic image 310. That is, the training data 350 may be generated by multiple labelling a plurality of pieces of data 360 including the plurality of determination values 331, 332, and 333 and the consensus value 340 on the diagnostic image 310. Herein, multi labelling may mean corresponding the respective determination values 331, 332, and 333 of the plurality of diagnosticians 321, 322, and 323 and the consensus value 340 for the same sign to one diagnostic image 310. In this case, the diagnostic image 310 may be training input data, and the plurality of determination values 331, 332, and 333 the consensus value 340 that are multi-labeled may be training result data.

FIG. 6 shows that the determination values 331, 332, and 333 and the consensus value 340 themselves are labeled on the diagnostic image 310, but no limitation thereto is imposed. The label values respectively corresponding to the determination values 331, 332, and 333 and the consensus value 340 may be labeled on the diagnostic image 310. For example, among the determination values and the consensus value, a value of 1 may be determined as a label value for a value determined as having a disease, a value of 0 may be determined as a label value for a value determined as not having a disease, and the label values may be labeled on the diagnostic image 310. In this case, when a first determination value is a value determined as having a disease and a second determination value is a value determined as not having a disease and a third determination value is a value determined as not having a disease and a consensus value is a value determined as having a disease, the values of [1, 0, 0, 1] may be labeled on the diagnostic image 310. However, specific label values and labelling methods are not limited thereto.

FIG. 6 shows that the diagnostic image 310 the same as the diagnostic image 310 used for diagnosis by the plurality of diagnosticians 321, 322, and 323 in FIGS. 4 and 5 is used as training data. However, no limitation thereto is imposed. The diagnostic image 310 used as training data may be a diagnostic image subjected to separate preprocessing. For example, a diagnostic image used by a plurality of diagnosticians for a disease diagnosis may be an image of a full face, but when a disease is an eye-related disease, a diagnostic image used as training data may be an image obtained by cropping the full-face image to leave an eye region. That is, in determining a state related to a disease by diagnosticians, a diagnostic image itself may be used for ease of determination. In generating a diagnostic model, separate preprocessing may be performed on a diagnostic image to exclude unnecessary determination factors in the diagnostic image, thereby increasing the accuracy of the generated diagnostic model.

In the meantime, FIG. 6 shows that the first to the third determination value 331, 332, and 333 and the consensus value 340 for one type of a disease and/or a sign are labeled on the diagnostic image 310, but no limitation thereto is imposed. Determination values and consensus values for at least two types of diseases and/or signs may be labeled on one diagnostic image. For example, a first to a third determination value and a first consensus value for a first disease, and a fourth to a sixth determination value and a second consensus value for a second disease may be multi-labeled on one diagnostic image. As another example, a first to a third determination value and a first consensus value for a first disease, a fourth to a sixth determination value and a second consensus value for a second disease, and a seventh to a ninth determination value and a third consensus value for a third disease may be multi-labeled on one diagnostic image. The specific number of diseases is not limited to the above-described range.

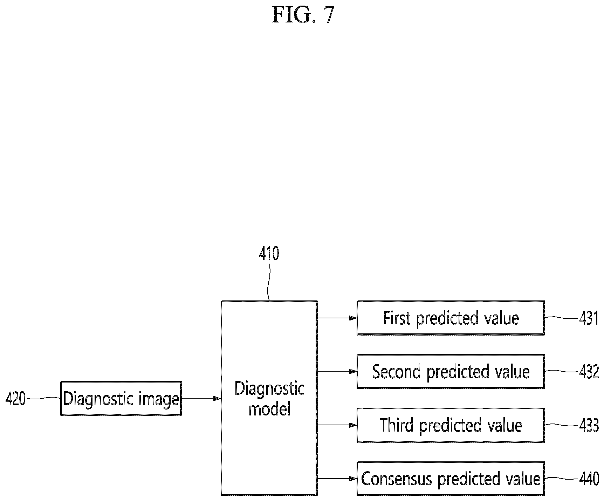

FIG. 7 is a diagram illustrating a process of predicting a disease by using a trained diagnostic model according to an embodiment.

Referring to FIG. 7, a diagnostic model 410 may be configured to output a plurality of predicted values 431, 432, 433, and 440 for an input diagnostic image 420. Specifically, the diagnostic model 410 may be configured to output, for the input diagnostic image 420, predicted values 431, 432 433 corresponding to respective determination values of a plurality of diagnosticians and a consensus predicted value 440 corresponding to a consensus value of the plurality of diagnosticians.

For example, the diagnostic model 410 may be
a model generated using algorithms, such as a convolution neural network (CNN), a vision transformer, a support vector machine (SVM), a random forest, a gradient boosting algorithm, an artificial neural network (ANN), a deep neural network (DNN), a recurrent neural network (RNN), ResNet, VGG, GoogLeNet, and/or MobileNet.

In the meantime, the diagnostic image 420 input to the diagnostic model 410 may be a diagnostic image subjected to separate preprocessing performed on a diagnostic image used as training data. That is, before a diagnostic image is input to the diagnostic model 410, preprocessing may be performed on the diagnostic image and the preprocessed diagnostic image may be input to the diagnostic model 410.

FIG. 7 shows that there are three predicted values 431, 432, and 433 corresponding to the respective determination values of the plurality of diagnosticians, and this is because there are three diagnosticians. If there are two or four or more diagnosticians, there may be two or four or more predicted values corresponding to determination values.

The diagnostic model 410 may be generated using the training data 350 described with reference to FIG. 6. In the training process, different weights for loss values may be set for respective output nodes. Specifically, in training the diagnostic model 410, the same loss weight may be set for all the plurality of output nodes. However, a high loss weight may be set for an output node corresponding to a consensus predicted value. For example, as shown in FIG. 7, when there are four output nodes, the loss weight for each of the output nodes may be set to 0.25. Alternatively, the loss weight for the node corresponding to the consensus predicted value 440 may be set to 0.7, and each of the loss weights for the nodes corresponding to the other predicted values 431, 432, and 433 may be set to 0.1. Alternatively, the loss weight for the node corresponding to the consensus predicted value 440 may be set to 0.5, and each of the loss weights for the nodes corresponding to the other predicted values 431, 432, and 433 may be set to 0.5/3. The specific values for the loss weights are not limited to the above-described values, and the specific values for the loss weights may be determined by appropriately adjusting the values through experimentation.

In the meantime, FIG. 7 shows that a first to a third predicted value 431, 432, and 433 and a consensus predicted value 440 for one type of a disease and/or a sign are output from the diagnostic model 410, but no limitation thereto is imposed. Predicted values and a consensus predicted value for each of at least two types of diseases and/or signs may be output from one diagnostic model. For example, when a diagnostic model is generated using a diagnostic image on which a first to a third determination value and a first consensus value for a first disease, and a fourth to a sixth determination value and a second consensus value for a second disease are multi-labeled, the diagnostic model may output predicted values and a consensus predicted value for each of the two types of diseases. As another example, when a diagnostic model is generated using a diagnostic image on which a first to a third determination value and a first consensus value for a first disease, a fourth to a sixth determination value and a second consensus value for a second disease, and a seventh to a ninth determination value and a third consensus value for a third disease are multi-labeled, the diagnostic model may output predicted values and a consensus predicted value for each of the three types of diseases. The specific number of diseases is not limited to the above-described range.

A final predicted value for determining the presence of a disease may be determined on the basis of the predicted values 431, 432, and 433 and the consensus predicted value 440 output by the diagnostic model 410. For example, among the output values, the consensus predicted value 440 may be determined as a final predicted value. Since one consensus predicted value 440 corresponding to a consensus value determined by a plurality of diagnosticians reaching an agreement is used as a final predicted value, the final predicted value may be obtained without an additional processing process for output values of the diagnostic model 410, thereby reducing accuracy loss due to the additional processing process.

In addition, a diagnostic model 410 according to an embodiment may learn more information than a model trained using only one determination value of each diagnostician or only one consensus value because respective determination values of a plurality of diagnosticians and a consensus value are learned in one model. Specifically, the determination values of the plurality of diagnosticians may influence the consensus value in the learning process, so the diagnostic model may learn more information, such as a definite positive, an ambiguous positive, an ambiguous negative, and a definite negative, than simply distinguishing between a positive and a negative. That is, the process of determining the respective determination values by the plurality of diagnosticians may be applied to a final predicted value, so prediction performance may be improved.

In addition, because respective determination values of a plurality of diagnosticians are learned in one model and the respective determination values may influence each other during the learning process, a diagnostic model 410 according to an embodiment may compensate for errors that some of the plurality of diagnosticians may cause during the process of determining the determination values, thereby increasing the accuracy of predicted values corresponding to the determination values.

In the meantime, a final predicted value for determining the presence of a disease and/or a sign may be determined on the basis of a value obtained by respectively applying weights to the predicted values 431, 432, and 433 and the consensus predicted value 440 output by the diagnostic model 410 and adding the values. For example, when the sum of the predicted values 431, 432, and 433 and the consensus predicted value 440 is equal to or greater than a threshold value, a positive may be determined. When the sum is less than the threshold value, a negative may be determined. In the meantime, in setting the weights, the weight for the consensus predicted value 440 may be set higher than the weights for the other predicted values 431, 432, and 433 and the values may be added.

In the meantime, a final predicted value for determining the presence of a disease may be determined by the majority of the predicted values 431, 432, and 433 and the consensus predicted value 440 output by the diagnostic model 410. For example, the final predicted value may be determined as a positive when the majority of all of the predicted values and the consensus predicted value are positive values, or the final predicted value may be determined as a negative when the majority are negative values. In the meantime, when the number of positive values is equal to the number of negative values, the final predicted value may be determined depending on the consensus predicted value.

In the meantime, a final predicted value for determining the presence of a disease may be determined on the basis of an average value of the predicted values 431, 432, and 433 and the consensus predicted value 440 output by the diagnostic model 410. Without being limited thereto, a final predicted value for determining the presence of a disease may be determined on the basis of other statistical values obtained using the predicted values 431, 432, and 433 and the consensus predicted value 440 output by the diagnostic model 410.

There may be various diagnostic models for predicting a disease and/or a sign on the basis of a diagnostic image, depending on the design purpose and the design method.

The inventors of the present application experimented the performance of a multi-label model according to the present disclosure by using a sign diagnostic model related to an eye disease. Specifically, the diagnostic image was an eye image of the user, the diagnostic subject was the eyelid, and the determination value was set to the presence of eyelid redness.

Three ophthalmology doctors were set as diagnosticians, and a comparative experiment was performed to experiment the performance of a first doctor model trained with only determination values of a first doctor, a second doctor model trained with only determination values of a second doctor, a third doctor model trained with only determination values of a third doctor, and a consensus value model trained with only consensus values of the first to third doctors.

Each model was generated using training data including 1020 diagnostic images, and the ResNet algorithm. Specifically, training data was randomly divided in the ratio train:validation:test = 7:1:2 and 30 iterations of the experiment were performed. That is, in one experiment, each model was generated using training data including 714 pieces of train data, 102 pieces of validation data, and 204 pieces of test data.

The F1-score, accuracy, sensitivity, specificity, PPV, and NPV of the models to be compared are shown in [Table 1] below.

**[Table 1]**

| | F1-score | Accuracy (%) | Sensitivity (%) | Specificity (%) | PPV (%) | NPV (%) |
|---|---|---|---|---|---|---|
| First doctor model | 0.6170 | 71.03 | 68.25 | 72.39 | 58.74 | 81.61 |
| Second doctor model | 0.6345 | 74.46 | 66.73 | 78.33 | 62.11 | 82.54 |
| Third doctor model | 0.6217 | 71.93 | 68.40 | 73.54 | 59.08 | 82.44 |
| Consensus value model | 0.6716 | 75.80 | 72.23 | 77.58 | 64.21 | 84.56 |

In the meantime, the performance of a multi-label model generated according to an embodiment of the present disclosure was experimented.

The multi-label model was generated using the ResNet algorithm and training data including 1020 diagnostic images on which the determination values of the first to the third doctor and the consensus values of the first to the third doctor were multi-labeled. Specifically, training data was randomly divided in the ratio train:validation:test = 7:1:2 and 30 iterations of the experiment were performed. That is, in one experiment, a multi-label model was generated using training data including 714 pieces of train data, 102 pieces of validation data, and 204 pieces of test data. The diagnostic images were the same as the diagnostic images used to generate each of the above-described models.

The F1-score, accuracy, sensitivity, specificity, PPV, and NPV of a first to a third predicted value and a consensus predicted value that were output values of a multi-label model generated according to an embodiment of the present disclosure are shown in [Table 2] below.

**[Table 2]**

| | F1-score | Accuracy (%) | Sensitivity (%) | Specificity (%) | PPV (%) | NPV (%) |
|---|---|---|---|---|---|---|
| First predicted value | 0.6171 | 72.66 | 64.01 | 77.13 | 60.84 | 80.35 |
| Second predicted value | 0.6445 | 74.46 | 69.03 | 77.22 | 61.77 | 83.28 |
| Third predicted value | 0.6239 | 73.61 | 64.54 | 78.23 | 62.59 | 81.24 |
| Consensus predicted value | 0.6817 | 76.29 | 73.80 | 77.57 | 64.80 | 85.25 |

Referring to [Table 1] and [Table 2], it can be seen that the performance of the diagnostic model generated using the training data in which the determination values of the first to the third diagnostician and the consensus value were multi-labeled on the diagnostic image was better than that of the models to be compared. In addition, it can be seen that the best performance was achieved when a final predicted value was determined on the basis of the consensus predicted value output by the multi-label model. In the meantime, it may be predicted that a similar trend will be shown even if the target disease of the eye disease diagnostic model experimented is changed to a different disease.

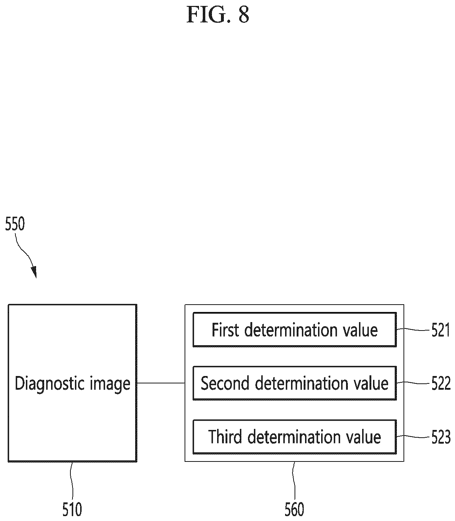

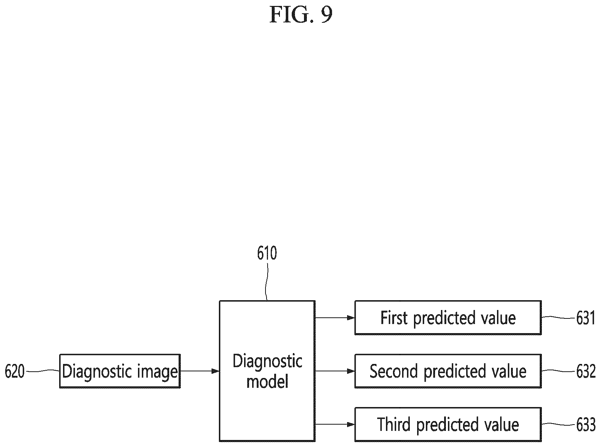

In the meantime, it has been described with reference to FIGS. 6 and 7 that the diagnostic model is generated using the training data in which the determination values of the plurality of diagnosticians and the consensus value are multi-labeled on the diagnostic image. However, as shown in FIGS. 8 and 9, a diagnostic model may be generated using training data in which only determination values of a plurality of diagnosticians are multi-labeled on a diagnostic image, excluding a consensus value. Even in this case, significant improvements as described above may still be achieved.

Specifically, FIG. 8 is a diagram illustrating training data used to train a diagnostic model according to an embodiment.

Referring to FIG. 8, training data 550 used to generate a diagnostic model may include a diagnostic image 510 and a plurality of determination values 521, 522, and 523 labeled on the diagnostic image 510. That is, the training data 550 may be generated by multiple labelling a plurality of pieces of data 560 on the diagnostic image 510. Herein, multi labelling may mean corresponding the respective determination values 331, 332, and 333 of the plurality of diagnosticians 321, 322, and 323 for the same sign to one diagnostic image 510. In this case, the diagnostic image 510 may be training input data, and the plurality of determination values 521, 522, and 523 that are multi-labeled may be training result data. The diagnostic image and the plurality of determination values have been described above, so a redundant description will be omitted.

In the meantime, FIG. 8 shows that the first to the third determination value 521, 522, and 523 for one type of a disease and/or a sign are labeled on the diagnostic image 510, but no limitation thereto is imposed. Determination values for at least two types of diseases and/or signs may be labeled on one diagnostic image, and the details thereof are similar to those described with reference to FIG. 6, so a redundant description will be omitted.

FIG. 9 is a diagram illustrating a process of predicting a disease by using a trained diagnostic model according to an embodiment.

Referring to FIG. 9, a diagnostic model 610 may be configured to output a plurality of predicted values 631, 632, and 633 for an input diagnostic image 620. Specifically, the diagnostic model 610 may be configured to output, for the input diagnostic image 620, the predicted values 631, 632, and 633 corresponding to respective determination values of a plurality of diagnosticians. The diagnostic model, the diagnostic image, and the predicted values have been described above, so a redundant description will be omitted.

In the meantime, FIG. 9 shows that a first to a third predicted value 631, 632, and 633 for one type of a disease and/or a sign are output from the diagnostic model 610, but no limitation thereto is imposed. Predicted values for each of at least two types of diseases and/or signs may be output from one diagnostic model, and the details thereof are similar to those described with reference to FIG. 7, so a redundant description will be omitted.

The inventors of the present application experimented the performance of a multi-label model using only determination values of diagnosticians according to the present disclosure by using a sign diagnostic model related to an eye disease. Specifically, the diagnostic image was an eye image of the user, the diagnostic subject was the lacrimal caruncle, and the determination values were set to the presence of lacrimal edema.

Three ophthalmology doctors were set as diagnosticians, and a comparative experiment was performed to experiment the performance of a first doctor model trained with only determination values of a first doctor, a second doctor model trained with only determination values of a second doctor, a third doctor model trained with only determination values of a third doctor, and the comparison between a value determined by the majority of the output values of the first to the third doctor model and a consensus value.

Each model was generated using training data including 1020 diagnostic images, and the ResNet algorithm. Specifically, training data was randomly divided in the ratio train:validation:test = 7:1:2 and 30 iterations of the experiment were performed. That is, in one experiment, each model was generated using training data including 714 pieces of train data, 102 pieces of validation data, and 204 pieces of test data.

The F1-score, accuracy, sensitivity, specificity, PPV, and NPV of experiments to be compared are shown in [Table 3] below.

**[Table 3]**

| | F1-score | Accuracy (%) | Sensitivity (%) | Specificity (%) | PPV (%) | NPV (%) |
|---|---|---|---|---|---|---|
| First doctor model | 0.4276 | 76.49 | 52.81 | 81.61 | 40.98 | 89.20 |
| Second doctor model | 0.4409 | 94.02 | 46.53 | 96.89 | 51.14 | 96.83 |
| Third doctor model | 0.4276 | 80.13 | 51.29 | 84.96 | 41.62 | 91.35 |
| Comparison between value determined by majority and consensus value | 0.4562 | 88.15 | 43.67 | 94.22 | 54.01 | 92.51 |

In the meantime, the performance of a multi-label model using only the determination values of the three doctors according to an embodiment of the present disclosure was experimented.

The multi-label model was generated using the ResNet algorithm and training data including 1020 diagnostic images on which the determination values of the first to the third doctor were multi-labeled. Specifically, training data was randomly divided in the ratio train:validation:test = 7:1:2 and 30 iterations of the experiment were performed. That is, in one experiment, a multi-label model was generated using training data including 714 pieces of train data, 102 pieces of validation data, and 204 pieces of test data. The diagnostic images were the same as the diagnostic images used to generate each of the above-described models.

The results of experiment with the performance of first to third predicted values that were output values of a multi-label model generated according to an embodiment of the present disclosure and the comparison between a value determined by the majority of the first to third predicted values and a consensus value are shown in [Table 4] below.

**[Table 4]**

| | F1-score | Accuracy (%) | Sensitivity (%) | Specificity (%) | PPV (%) | NPV (%) |
|---|---|---|---|---|---|---|
| First predicted value | 0.4427 | 78.59 | 48.61 | 85.37 | 45.14 | 88.52 |
| Second predicted value | 0.4328 | 93.40 | 43.84 | 96.36 | 55.58 | 96.73 |
| Third predicted value | 0.4459 | 81.93 | 49.39 | 87.40 | 44.31 | 91.14 |
| Comparison between value determined by majority and consensus value | 0.4814 | 86.70 | 52.40 | 91.37 | 49.96 | 93.49 |

Referring to [Table 3] and [Table 4], it can be seen that the performance of the diagnostic model generated using the training data in which the determination values of the plurality of diagnosticians were multi-labeled was better than that of the models to be compared on average. That is, it can be seen that significant improvement may be achieved by generating the diagnostic model using the training data in which the determination values of the plurality of diagnosticians are multi-labelled on the diagnostic image.

### 5. Diagnostic model 2 - label smoothing model

Hereinafter, the specific details of a label smoothing model for diagnosing a disease and/or a sign will be described.

A label smoothing model according to an embodiment may be a model that uses a label value for a consensus value determined by a plurality of diagnosticians for one diagnostic image, and is generated using training data in which smoothing is performed on the label value considering a plurality of determination values respectively determined by the plurality of diagnosticians, and the smoothed label value is labeled on the diagnostic image.

The diagnostic image, the diagnostic subjects, the diagnosticians, the determination values, the consensus values, and the label values have been described above in the section describing the multi-label model, so a redundant description will be omitted.

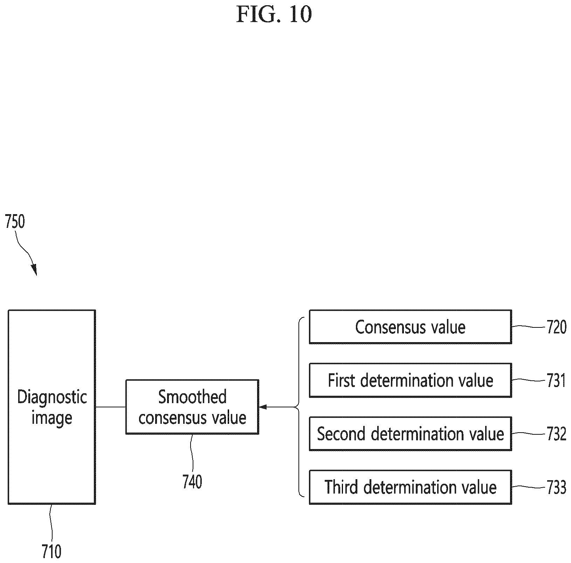

Training data used to generate a diagnostic model will be described with reference to FIG. 10.

FIG. 10 is a diagram illustrating training data used to train a diagnostic model according to an embodiment.

Referring to FIG. 10, first, a plurality of diagnosticians may diagnose a state related to a disease and/or a sign for the same diagnostic image 710 independently of each other to determine determination values 731, 732, and 733 for the respective diagnosticians. In addition, the plurality of diagnosticians may determine one consensus value 720 by reaching a mutual agreement about a state related to a disease and/or a sign for the same diagnostic image 710.

The plurality of diagnosticians who determine the plurality of determination values 731, 732, and 733 may be the same as the plurality of diagnosticians who determine the consensus value 720, and the diagnostic image 710 used to determine the plurality of determination values 731, 732, and 733 may be the same as the diagnostic image 710 used to determine the consensus value 720, and the diagnostic image 710 used as training data may be a diagnostic image subjected to separate preprocessing. This has been described above, so a redundant description will be omitted.

Referring to FIG. 10, a label value labeled on the diagnostic image 710 may be a smoothed consensus value 740 determined on the basis of the plurality of determination values 731, 732, and 733 and the consensus value 720. For example, when the consensus value 720 is a positive and the determination values 731, 732, and 733 determined by the plurality of diagnosticians independently of each other are unanimously positive, the smoothed consensus value 740 may be determined to be a value of 1. When the consensus value 720 is a negative and the determination values 731, 732, and 733 determined by the plurality of diagnosticians independently of each other are unanimously negative, the smoothed consensus value 740 may be determined to be a value of 0. When the consensus value 720 is a positive and the plurality of determination values 731, 732, and 733 are not unanimous, the smoothed consensus value 740 may be determined to be a value of 0.8. When the consensus value 720 is a negative and the plurality of determination values 731, 732, and 733 are not unanimous, the smoothed consensus value 740 may be determined to be a value of 0.2. Without being limited thereto, when the consensus value 720 is a positive and the plurality of determination values 731, 732, and 733 are not unanimous, the smoothed consensus value 740 may be determined to a value of 0.7. In the meantime, when the consensus value 720 is a negative and the plurality of determination values 731, 732, and 733 are not unanimous, the smoothed consensus value 740 may be determined to be a value of 0.3. That is, the smoothed consensus value 740 may be determined by appropriately adjusting the consensus value 730 considering the distribution of the plurality of determination values 720, and is not limited to the values described above.

In the meantime, FIG. 10 shows three determination values 720, that is, the case in which there are three diagnosticians, but no limitation thereto is imposed, and there may be two or four or more diagnosticians. In this case, the number of determination values 720 may be two, or four or more.

In the meantime, FIG. 10 shows that the smoothed consensus value 740 determined on the basis of the consensus value 720 and the first to the third determination value 731, 732, and 733 for one type of a disease and/or a sign are labeled on the diagnostic image 710, but no limitation thereto is imposed. At least two smoothed consensus values determined on the basis of determination values and a consensus value for each of at least two types of diseases and/or signs may be labeled on one diagnostic image. For example, a first smoothed consensus value determined on the basis of a first consensus value and a first to a third determination value for a first disease, and a second smoothed consensus value determined on the basis of a second consensus value and a fourth to a sixth determination value for a second disease may be multi-labeled on one diagnostic image. As another example, a first smoothed consensus value determined on the basis of a first consensus value and a first to a third determination value for a first disease, a second smoothed consensus value determined on the basis of a second consensus value and a fourth to a sixth determination value for a second disease, and a third smoothed consensus value determined on the basis of a third consensus value and a seventh to a ninth determination value for a third disease may be multi-labeled on one diagnostic image. The specific number of diseases is not limited to the above-described range.

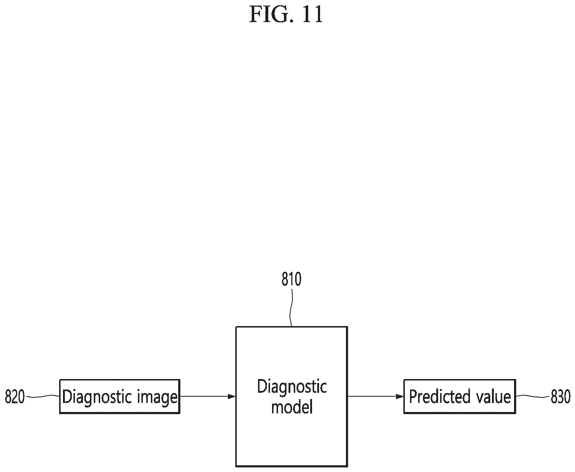

FIG. 11 is a diagram illustrating a process of predicting a disease by using a trained diagnostic model according to an embodiment.

Referring to FIG. 11, a diagnostic model 810 may be configured to output a predicted value 830 corresponding to a smoothed consensus value for an input diagnostic image 820.

For example, the diagnostic model 810 may be a model generated using algorithms, such as a convolution neural network (CNN), a vision transformer, a support vector machine (SVM), a random forest, a gradient boosting algorithm, an artificial neural network (ANN), a deep neural network (DNN), a recurrent neural network (RNN), ResNet, VGG, GoogLeNet, and/or MobileNet.

In the meantime, it has been described above that the diagnostic image 820 input to the diagnostic model 810 may be a diagnostic image subjected to separate preprocessing performed on a diagnostic image used as training data, so a redundant description will be omitted.

In the meantime, FIG. 11 shows that the predicted value 830 for one type of a disease and/or a sign is output from the diagnostic model 810, but no limitation thereto is imposed. A predicted value for each of at least two types of diseases and/or signs may be output from one diagnostic model. For example, when a diagnostic model is generated using a diagnostic image on which a first smoothed consensus value determined on the basis of a first consensus value and a first to a third determination value for a first disease, and a second smoothed consensus value determined on the basis of a second consensus value and a fourth to a sixth determination value for a second disease are multi-labeled, the diagnostic model may output a predicted value for each of the two types of diseases. As another example, when a diagnostic model is generated using a diagnostic image on which a first smoothed consensus value determined on the basis of a first consensus value and a first to a third determination value for a first disease, a second smoothed consensus value determined on the basis of a second consensus value and a fourth to a sixth determination value for a second disease, and a third smoothed consensus value determined on the basis of a third consensus value and a seventh to a ninth determination value for a third disease are multi-labeled, the diagnostic model may output a predicted value for each of the three types of diseases. The specific number of diseases is not limited to the above-described range.

A final predicted value for determining the presence of a disease may be determined on the basis of the predicted value 830 output by the diagnostic model 810. Since the predicted value 830 corresponding to the smoothed consensus value determined considering the determination values of the plurality of diagnosticians and the consensus value is used as a final predicted value, the final predicted value may be obtained without an additional processing process for output values of the diagnostic model 810, thereby reducing accuracy loss due to the additional processing process.

In addition, the predicted value 830 obtained from the diagnostic model 810 is a predicted value corresponding to a smoothed consensus value to which the determination values of the plurality of diagnosticians are applied, so the final predicted value may reflect the determination values of the plurality of diagnosticians.

There may be various diagnostic models for predicting a disease and/or a sign on the basis of a diagnostic image, depending on the design purpose and the design method.

The inventors of the present application experimented the performance of a label smoothing model according to the present disclosure by using a sign diagnostic model related to an eye disease. Specifically, the diagnostic image was an eye image of the user, the diagnostic subject was the eyelid, and the determination value was set to the presence of eyelid redness.

Three ophthalmology doctors were set as diagnosticians, and the performance of a first doctor model trained with only determination values of a first doctor, a second doctor model trained with only determination values of a second doctor, a third doctor model trained with only determination values of a third doctor, and a consensus value model trained with only consensus values of the first to third doctors was experimented through a comparative experiment.

Each model was generated using training data including 1020 diagnostic images, and the ResNet algorithm. Specifically, training data was randomly divided in the ratio train:validation:test = 7:1:2 and 30 iterations of the experiment were performed. That is, in one experiment, each model was generated using training data including 714 pieces of train data, 102 pieces of validation data, and 204 pieces of test data.

The F1-score, accuracy, sensitivity, specificity, PPV, and NPV of the models to be compared are shown in [Table 5] below. This is the same experimental result as in [Table 1].

**[Table 5]**

| | F1-score | Accuracy (%) | Sensitivity (%) | Specificity (%) | PPV (%) | NPV (%) |
|---|---|---|---|---|---|---|
| First doctor model | 0.6170 | 71.03 | 68.25 | 72.39 | 58.74 | 81.61 |
| Second doctor model | 0.6345 | 74.46 | 66.73 | 78.33 | 62.11 | 82.54 |
| Third doctor model | 0.6217 | 71.93 | 68.40 | 73.54 | 59.08 | 82.44 |
| Consensus value model | 0.6716 | 75.80 | 72.23 | 77.58 | 64.21 | 84.56 |

In the meantime, the performance of a label smoothing model according to an embodiment of the present disclosure was experimented.

The label smoothing model was generated using the ResNet algorithm and training data including 1020 diagnostic images on which on the basis of the consensus value determined by the first to the third doctor reaching an agreement and the determination values respectively determined by the first to the third doctor, a value of 1, 0.8, 0.2, or 0 were multi-labeled as the smoothed consensus value. Specifically, training data was randomly divided in the ratio train:validation:test = 7:1:2 and 30 iterations of the experiment were performed. That is, in one experiment, a multi-label model was generated using training data including 714 pieces of train data, 102 pieces of validation data, and 204 pieces of test data. The diagnostic images were the same as the diagnostic images used to generate each of the above-described models.

The F1-score, accuracy, sensitivity, specificity, PPV, and NPV of a predicted value output by a label smoothing model generated according to an embodiment of the present disclosure are shown in [Table 6] below.

**[Table 6]**

| | F1-score | Accuracy (%) | Sensitivity (%) | Specificity (%) | PPV (%) | NPV (%) |
|---|---|---|---|---|---|---|
| Predicted value | 0.6893 | 76.54 | 75.85 | 76.83 | 64.69 | 86.21 |

Referring to [Table 5] and [Table 6], it can be seen that the performance of the label smoothing model was better than that of the models to be compared. In the meantime, it may be predicted that a similar trend will be shown even if the target disease of the eye disease diagnostic model experimented is changed to a different disease.

### 6. A system for predicting a clinical activity score (CAS) for thyroid eye disease

Hereinafter, a system for predicting a clinical activity score for a thyroid eye disease by using the above-described diagnostic model will be described in detail.

A clinical activity score for thyroid eye disease may be determined considering a total of seven items. Specifically, the seven items include conjunctival hyperemia (redness of conjunctiva), conjunctival edema (swelling of conjunctiva), lacrimal edema (swelling of lacrimal caruncle), eyelid redness (redness of eyelid), eyelid edema (swelling of eyelid), spontaneous retrobulbar pain, and pain on an attempted upward or downward gaze. Each sign is assigned a score of 1 for positive, or a score of 0 for negative, and all the scores are added to determine the final clinical activity score.

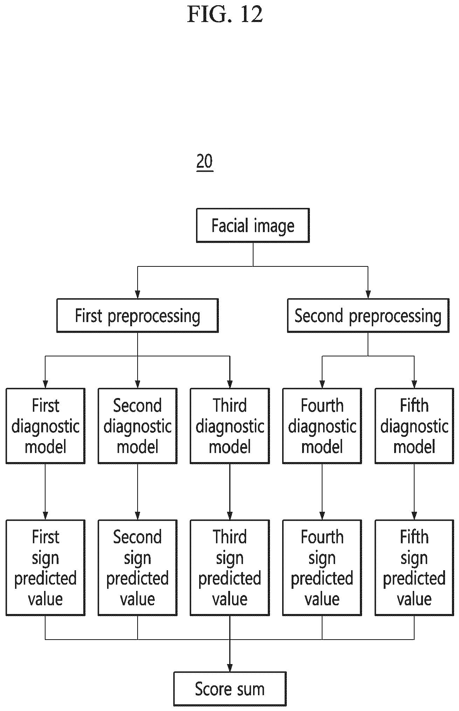

FIG. 12 is a diagram illustrating a system 20 for predicting a clinical activity score for thyroid eye disease according to an embodiment.

The system 20 may obtain, from a user, information on spontaneous retrobulbar pain and pain on an attempted upward or downward gaze among a total of seven items considered in determining a clinical activity score for thyroid eye disease. Specifically, although not shown in FIG. 12, but the system 20 displays a graphical user interface (GUI) for receiving user input on a display of a user device, and when a user inputs information into the user device, the system 20 may obtain information on each sign from the user device.

The system 20 may use independent diagnostic models to perform prediction for conjunctival hyperemia (redness of conjunctiva), conjunctival edema (swelling of conjunctiva), lacrimal edema (swelling of lacrimal caruncle), eyelid redness (redness of eyelid), and eyelid edema (swelling of eyelid) in a clinical activity score for thyroid eye disease. Specifically, a facial image representing the user's eye obtained from the user device and five types of diagnostic models for predicting the respective signs may be used to predict a score for each of the five types of signs.

To predict a score for each sign, the system 20 may first obtain a facial image and/or an eye image representing the user's eye. The eye image may mean an image representing the conjunctiva, the white of the eye, the cornea, the iris, the eyelid, the eyebrow, the eyelashes, the eyeball exposed to the outside, the outline of the eye, the cornea exposed to the outside, and the conjunctiva exposed to the outside. Alternatively, the eye image may mean an image about the user's upper eyelid, lower eyelid, and the eyeball (exposed eyeball) exposed to the outside by the upper eyelid and the lower eyelid.

The user uses the user device to photograph his or her face in person to obtain a facial image, and the user device transmits the facial image to the system 20, thereby obtaining the eye image.

For example, the user may use the user device to obtain his or her facial image, and the user device may perform preprocessing on the facial image to obtain an eye image, and the user device may transmit the eye image to the system 20, and the system 20 may obtain the eye image. As another example, the user may use the user device to obtain his or her facial image, and the user device may transmit the facial image to the system 20, and the system 20 may perform preprocessing on the facial image to obtain an eye image. Without being limited thereto, the user may use the user device to directly obtain his or her eye image, and the user device may transmit the eye image to the system 20.

The user device may provide a photographing guide to enable the user to easily obtain a facial image. The photographing guide may be displayed on the display of the user device or may be provided to the user by voice and/or sound. For example, the user device may display the facial outline and/or a desired eye position on a preview image obtained by photographing the user, thus guiding the user in adjusting the photographing angle, position, and/or direction of the user device easily and intuitively.

The system 20 may use a first to a fifth diagnostic model to obtain a predicted value for each of the five types of signs. Specifically, the first to the fifth diagnostic model may be a conjunctival hyperemia diagnostic model, a conjunctival edema diagnostic model, a lacrimal edema diagnostic model, an eyelid edema diagnostic model, and an eyelid redness diagnostic model, respectively.

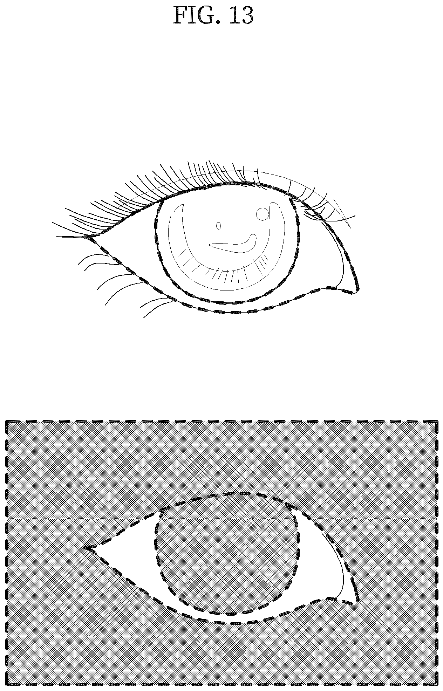

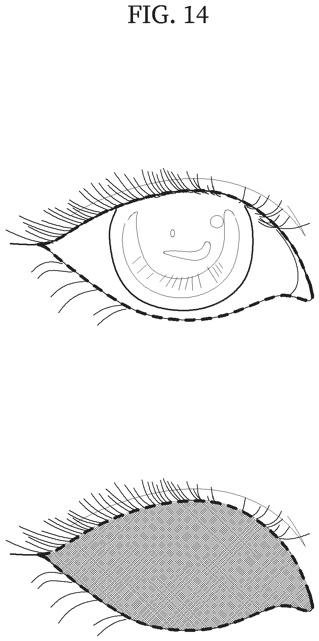

Before inputting the obtained facial image to the first to the fifth diagnostic model, the system 20 may perform two different types of preprocessing on the facial image. The specific details of preprocessing will be described with reference to FIGS. 13 and 14.

FIGS. 13 and 14 are diagrams illustrating an image preprocessing process according to an embodiment.

The system 20 may perform first preprocessing on an image to be input to the models for diagnosing conjunctival hyperemia, conjunctival edema, and lacrimal edema, wherein an eyeball region is the main subject for determination. Referring to FIG. 13, the first preprocessing may mean preprocessing to mask the pupil and the skin, and the preprocessed image may be an eyeball region image.

The system 20 may perform second preprocessing on an image to be input to the models for diagnosing eyelid edema and eyelid redness, wherein an eyelid region is the main subject for determination. Referring to FIG. 14, the second preprocessing may mean preprocessing to mask the eyeball region, and the preprocessed image may be an eyelid region image.

The system 20 may input the respective preprocessed images to the first to the fifth diagnostic model to obtain a first to a fifth sign predicted value. The system 20 may obtain, on the basis of the first to the fifth sign predicted value, scores for conjunctival hyperemia, conjunctival edema, lacrimal edema, eyelid redness, and eyelid edema, and may add the obtained scores. The system 20 may further add the scores for spontaneous retrobulbar pain and pain on an attempted upward or downward gaze obtained from the user to obtain a clinical activity score for thyroid eye disease.

The system 20 may transmit the obtained clinical activity score and/or information on thyroid eye disease to the user device and/or other external devices. For example, the system 20 may transmit a message indicating that the user is at risk of thyroid eye disease to the user device when the obtained clinical activity score is equal to or greater than a reference value. Additionally, the system 20 may transmit a message for guiding the user to visit a hospital due to risk of thyroid eye disease, to the user device. The user device may provide a message received from the system 20 to the user through the user interface.

The first to the fifth diagnostic model used by the system 20 are models for diagnosing signs using images, and the first to the fifth diagnostic model may be realized as multi-label models and/or label smoothing models described above. Without being limited thereto, the first to the fifth diagnostic model may be realized as various types of models for outputting predicted values on the basis of images. In addition, the first to the fifth diagnostic model may not all be generated with the same algorithm. That is, at least some of the first to the fifth diagnostic may be models generated by different algorithms, or all of the models may be models generated by different algorithms.

In the meantime, how to determine training data and/or evaluation data to use a diagnostic model for actual diagnosis is a factor that needs to be considered in order to safely use the diagnostic model. Specifically, the performance of a diagnostic model based on evaluation data needs to be equal to or greater than a standard, so that the diagnostic model is used for actual diagnosis. Therefore, the determination of the evaluation data may require a high standard.

Accordingly, determination values and/or a consensus value included in evaluation data for evaluating the performance of a diagnostic model may be determination values and/or a consensus value that are determined by diagnosticians actually meeting and diagnosing a patient. A diagnostic image included in evaluation data may be obtained from a patient after diagnosticians actually meet and diagnose the patient or before diagnosticians meet the patient. That is, determination values and/or a consensus value to be labeled on a diagnostic image that is evaluation data may be values determined by a plurality of diagnosticians meeting and diagnosing an actual patient, rather than values determined through the diagnostic image. The details related to the diagnosticians meeting a user in person and diagnosing the user to determine determination values and a consensus value have been described above, so a redundant description will be omitted.

Determination values and/or a consensus value included in training data to be used to train a diagnostic model may also be determination values and/or a consensus value determined by diagnosticians actually meeting and diagnosing a patient as described above.

In the meantime, training data may not necessarily require a high standard as evaluation data. Accordingly, training data may include determination values and/or a consensus value determined by diagnosticians diagnosing an image of a patient, rather than an actual patient. Alternatively, determination values included in training data may be values determined by diagnosticians diagnosing a patient through an image of the patient, and a consensus value may be a value determined by the majority of the determination values.

Various substitutions, modifications, and changes from the spirit of the present disclosure defined in the following claims by those skilled in the art are also included in the scope of the present disclosure, so that the present disclosure described above is not limited to the embodiments and the accompanying drawings. In addition, the embodiments described herein are not applied in a limited manner, and all or some of the embodiments may be selectively combined so that various modifications can be made. Further, steps constituting each embodiment may be used individually or in combination with steps constituting other embodiments.

### Mode for Invention

-

## Claims

1. A sign prediction method, comprising:
obtaining a facial image including eye region;
obtaining a plurality of result values using the facial image and a diagnostic model for predicting an eye-related sign, wherein the plurality of result values comprises a plurality of predicted values and a single consensus predicted value; and
determining a presence of the eye-related sign based on the consensus predicted value among the plurality of result values,
wherein the diagnostic model is generated using training data in which a plurality of determination values, independently determined by two or more different diagnosticians with respect to a presence of the eye-related sign in a same diagnostic subject, and a single consensus value, determined by the diagnosticians through mutual agreement with respect to a presence of the eye-related sign in the same diagnostic subject, are multi-labeled on one diagnostic image,
wherein the plurality of predicted values corresponds to the plurality of determination values, and the consensus predicted value corresponds to the consensus value,
wherein the eye-related sign is at least one of a conjunctival hyperemia, a conjunctival edema, a lacrimal edema, an eyelid redness and an eyelid edema.

2. The method of claim 1,
wherein at least some of the training data have the consensus value corresponding to a minority of the determination values among the plurality of determination values.

3. The method of claim 1,
wherein, when training the diagnostic model, a loss weight for a node where the consensus predicted value is output is set higher than a loss weight for each node where the predicted values are output.

4. The method of claim 1,
wherein obtaining the plurality of result values comprises:
performing preprocessing on the facial image for the eye region; and
obtaining the plurality of result values using the preprocessed image and the diagnostic model.

5. The method of claim 4,
wherein performing preprocessing comprises:
when the eye-related sign is at least one of the conjunctival hyperemia, the conjunctival edema, and the lacrimal edema, performing preprocessing on the facial image to mask a pupil and skin; and
when the eye-related sign is at least one of the eyelid redness and the eyelid edema, performing preprocessing on the facial image to mask an eyeball region.

6. The method of claim 1,
wherein the diagnosticians are three ophthalmologists with over 15 years of experience,
wherein the determination values comprise three values corresponding to each diagnostician.

7. The method of claim 1,
wherein at least one of the determination values and the consensus value is a value determined by the diagnosticians through actually meeting with the diagnostic subject with respect to the presence of the eye-related sign.

8. The method of claim 1,
wherein at least one of the determination values and the consensus value is a value determined by the diagnosticians through a facial image of the diagnostic subject with respect to the presence of the eye-related sign.

9. The method of claim 1,
wherein the consensus value is determined after the diagnosticians determine each of the determination values.

10. The method of claim 1,
wherein the diagnostic model is generated using at least one algorithm of a convolution neural network (CNN), a vision transformer, a support vector machine (SVM), a random forest, a gradient boosting algorithm, an artificial neural network (ANN), a deep neural network (DNN), a recurrent neural network (RNN), ResNet, VGG, GoogLeNet, and MobileNet.
